# NEUE EUROPÄISCHE PATENTSCHRIFT

(11) **EP 1 037 647 B2**
(45) Veröffentlichungstag und Bekanntmachung des Hinweises auf die Entscheidung über den Einspruch: **15.06.2005**
(45) Hinweis auf die Patenterteilung: 08.08.2001
(21) Anmeldenummer: 98966628.4
(22) Anmeldetag: 16.12.1998
(51) Int. Cl.: A61K 35/78

(54) **ARZNEIPFLANZENTROCKENEXTRAKTE**
MEDICINAL PLANT DRY EXTRACTS
EXTRAITS SECS DE PLANTE MEDICINALE

(30) Priorität: 19.12.1997 DE 19756677; 28.03.1998 DE 19814014
(43) Veröffentlichungstag der Anmeldung: 27.09.2000
(73) Patentinhaber: Krewel Meuselbach GmbH, 53783 Eitorf (DE)
(72) Erfinder: SCHIERSTEDT, Detlef, D-53757 St. Augustin (DE)
(74) Vertreter: Jönsson, Hans-Peter, Dr.Dipl.-Chem.
(86) Internationale Anmeldenummer: PCT/EP1998/008247
(87) Internationale Veröffentlichungsnummer: WO 1999/032130

(56) Entgegenhaltungen:
- EP-A- 0 265 338
- EP-A- 0 496 705
- EP-A- 0 664 131
- EP-A- 0 702 957
- EP-A- 0 722 719
- WO-A-90/03179
- DE-C- 4 201 172

## Beschreibung

Gegenstand der Erfindung sind peroral applizierbare Arzneipflanzentrockenextrakte. Die EP 0 702 957 A betrifft peroral applizierbare Johanniskrautextrakte sowie ein Verfahren zu ihrer Herstellung. Johanniskrautfluidextrakten wird Polyvinylpyrrolidon zugegeben und die Fluidphase bei erhöhter Temperatur und/oder unter Vakuum bis zur Trockne **eingeengt**. Hieraus resultiert eine verbesserte Freisetzung der Dianthrone gegenüber Standardpräparaten.

Die EP 0 664 131 A betrifft peroral applizierbare Kava-Fluidextrakte sowie ein Verfahren zu ihrer Herstellung. Kava-Fluidextrakte werden mit einem bei Raumtemperatur festen Träger, ausgewählt aus Polyvinylpyrrolidon, Cellulosederivaten und/oder Stärkederivaten in Kontakt gebracht und der Fluidextrakt bei erhöhter Temperatur und/oder unter Vakuum bis zur Trockne eingeengt. Hieraus resultiert eine verbesserte Freisetzung der 6 Hauptkavapyrone gegenüber Standardpräparaten.

Die medizinische Wirkung von Arzneipflanzentrockenextrakten aus Sabalfructus, Passiflora, Agnus castus, Crataegus, Kava, Gingko, Brennessel, Mariendistel und/oder Hypericum ist aus dem Stand der Technik bekannt.

Die DE 42 01 172 C1 betrifft Aloe Vera-Extrakt enthaltende Pellets, die durch eine Dispersion des Aloe Vera-Extrakts in einer Matrix, die vorwiegend aus einem Gerüstbildner, nämlich Kollagen, Gelatine, fraktionierter Gelatine, einem Kollagenhydrolysat und/oder einem Gelatinederivat besteht, gebildet werden.

Üblichen, insbesondere im Handel erhältliche Trockenextrakten ist jedoch gemein, daß die Wirkstofffreisetzung, d. h. die Geschwindigkeit und der Grad der Freisetzung der Inhaltsstoffe, bedingt durch den Naturstoffcharakter der Arzneipflanzen, stark von Charge zu Charge variiert. Selbst Wettbewerbsprodukte mit gleichen Extraktdeklarationen weichen oft erheblich voneinander ab. Auch die bekannten Arzneibücher definieren lediglich die Menge der Inhaltsstoffe, aber nicht Eigenschaften, die für eine zuverlässige Therapie mit den Extrakten wünschenswert wären.

Die Aufgabe der vorliegenden Erfindung besteht daher darin, Arzneipfianzentrockenextrakte sowohl im Grad als in der Geschwindigkeit auf hohem Niveau zu standardisieren und somit die Therapiesicherheit zu erhöhen.

In einem ersten Aspekt der vorliegenden Erfindung wird die vorgenannte Aufgabe gelöst durch peroral applizierbare Arzneipflanzentrockenextrakte gemäß Anspruch 1.

Bei den Naturstoffextrakten, die eine Vielzahl von wirksamen Bestandteilen und weiteren Begleitstoffen enthalten, war es nicht zu erwarten, daß ein derart inhomogenes Substanzgemisch eine hohe Löslichkeitsverbesserung in den genannten Trägern und dadurch eine Standardisierung der Freisetzung mit sich bringt.

Überraschenderweise wurde gefunden, daß durch Lösen der Trägermaterialien in einem Fluidextrakt nach dem Einengen ein Extrakt-Träger-Komplex entsteht, bei dem die wirksamen Bestandteile in mikrodisperser Form und/oder in einer halbfesten oder festen Lösung so gebunden sind, daß die Geschwindigkeit und der Grad der Freisetzung der wirksamen Bestandteile, gegebenenfalls durch Vermischen verschiedener Chargen, standardisiert werden konnte. Bei der halbfesten oder festen Extraktphase handelt es sich insbesondere um eine Spissum- oder Siccumphase, die die wirksamen Bestandteile enthält.

Die Wirkstoffreisetzung aus festen peroralen Arzneiformen kann beispielsweise nach dem Deutschen Arzneibuch DAB 10 V5.4 bestimmt werden. Bei Messungen der Freisetzung der Extrakte unter Einsatz eines Mediums, das dem Magen-Milieu entspricht, wurde eine gegenüber dem Stand der Technik signifikant verbesserte Freisetzung der wirksamen Bestandteile erreicht.

Die alkohollöslichen oder mit Alkohol mischbaren, wasserlöslichen oder mit Wasser mischbaren, bei Raumtemperatur (25°C) halbfesten oder festen Träger sind ausgewählt aus Stoffen, die dadurch gekennzeichnet sind, daß der Träger ausgewählt ist aus Polyethylenglykolen, insbesondere mit einer Molmasse von wenigstens 1000, Polyvinylalkoholen, Polyvidonacetat und/oder Polyvinylpyrrolidon.

Polyvinylalkohol im Sinne der vorliegenden Erfindung weist üblicherweise eine Molmasse von 28000 bis 40000 auf. Polyethylenglykole mit entsprechenden Molmassen von wenigstens 1000, beispielsweise 4000 sind im Handel erhältlich. Die Molmasse bestimmt dabei die Konsistenz bei Raumtemperatur. Polyvinylpyrrolidone sind beispielsweise unter der Bezeichnung "Kollidon®" mit unterschiedlicher Molmasse im Handel erhältlich. Gleiches gilt für Polyvidonacetate, die unter der Bezeichnung "Kollidon VA" erhältlich sind.

Träger II ist ausgewählt aus alkoholunlöslichen, wasserunlöslichen, in Wasser quellbaren, bei Raumtemperatur festen Trägern oder Erdalkalimetall- und/oder Alkalimetall-Carbonaten einschließlich Hydrogen-Carbonaten.

Bevorzugt wird der Träger II ausgewählt aus Carbonaten, wie Natriumhydrogencarbonat, Calciumcarbonat oder Magnesiumcarbonat sowie mit Wasser quellbaren organischen Polymeren, insbesondere Stärke, Stärkederivate, Cellulose, Cellulosederivate, wobei hier quervernetzte Cellulosederivate besonders bevorzugt sind. In gleicher Weise sind besonders bevorzugte Träger II quervernetzte Polyvinylpyrrolidonderivate, die beispielsweise unter der Bezeichnung Kollidon®Cl oder Polyplastone im Handel erhältlich sind. Unter Verwendung von an sich im Stand der Technik bekannten Sprengmitteln oder Brauseagenzien lassen sich somit auch Brausetabletten oder Trinktabletten herstellen.

Besonders bevorzugt im Sinne der vorliegenden Erfindung beträgt das Gewichtsverhältnis von Träger II zu Träger I 30 : 1 bis 1 : 1, besonders bevorzugt 10 : 1 bis 3 : 1.

Im Sinne der vorliegenden Erfindung sind die Arzneipflanzen ausgewählt aus Passiflora, Agnus castus, Crataegus, Gingko, Brennessel, Baldrian, **Cimicifuga-Wurzel** oder Wurzelstock und/oder Cynara.

Wenn im Sinne der vorliegenden Erfindung der Begriff "alkohollöslich" verwendet wird, so ist darunter eine Löslichkeit, gegebenenfalls unter Erwärmen in Methanol, Ethanol oder 2-Propanol zu verstehen. Mit diesen Lösungsmitteln werden häufig Fluidextrakte hergestellt. Daneben ist aber auch die Extraktion mit anderen Lösungsmitteln wie Aceton, Aceton-Wassergemischen oder die Extraktion mit überkritischem Dampf oder Kohlendioxid bekannt. Der Begriff der Löslichkeit ist in verschiedenen Arzneibüchern unterschiedlich definiert. Im Sinne der vorliegenden Erfindung gilt ein Träger als löslich, wenn sich wenigstens 15 Gew.-% des Trägers, gegebenenfalls unter Erwärmen in dem Lösungsmittel bei Raumtemperatur praktisch vollständig löst.

Mit Hilfe der vorliegenden Erfindung ist es möglich, standardisierte Trockenextrakte mit definierter Freisetzung zur Verfügung zu stellen, bei denen das Gewichtsverhältnis von nichtflüchtiger Extraktphase zu Träger vorzugsweise im Bereich von 1 : 10 bis 1 : 0,25, insbesondere 1 : 5 bis 1 : 0,6, besonders bevorzugt 1 : 3 bis 1 : 0,7 beträgt.

Wird das Verhältnis von Extrakt zu Träger zu groß gewählt, so ist die Bindung des Extraktes an Träger nicht ausreichend und damit keine optimale Freisetzung der wirksamen Bestandteile gegeben.

Zur Herstellung von Tabletten, Brausetabletten, Filmtabletten, Dragees, Trinktabletten, Granulate oder Kapseln werden die Arzneipflanzentrockenextrakte oder deren Vorläufer, die Fluidextrakte, gegebenenfalls mit an sich bekannten weiteren Hilfs- und/oder Zusatzstoffen versetzt. So können bei der Herstellung von Tabletten die Hilfs- und/oder Zusatzstoffe vorzugsweise ausgewählt sein aus Bindemitteln, Gleitmitteln, Füllstoffen, Emulgatoren, Netzmitteln, Übergangsmitteln und/oder Sprengmitteln.

Zum Einsatz kommen vorzugsweise Fluidextrakte. Zu diesen wird das Trägermaterial, gegebenenfalls bei erhöhter Temperatur, zugegeben. Bei erhöhter Temperatur und/oder unter Vakuum wird der Fluidextrakt anschließend bis zur Trockne eingeengt. Fluidextrakte umfassen auch Spissum- oder durch geeignete Lösungsmittel wieder ganz oder teilweise gelöste Trockenextrakte, die nach an sich bekannten Verfahren hergestellt werden.

Das Einengen zur Trockne kann beispielsweise auch durch Sprühtrocknung nach bekannten Verfahren und mit üblichen technischen Geräten, wie Sprühtrocknern oder Wirbelschichtgranulatoren erfolgen.

Die Fluidextrakte können auf einfache Weise dadurch hergestellt werden, daß die Arzneimittelpflanzen oder die entsprechenden Pflanzenteile in einem geeigneten Lösungsmittel extrahiert werden. Vorzugsweise wird das Lösungsmittel ausgewählt aus Aceton, Chloroform, Ethylacetat und C₁-C₄-Alkoholen, deren Gemischen untereinander und mit Wasser. Besonders bevorzugte Lösungsmittel sind Methanol, Ethanol, und 2-Propanol sowie deren wasserfreie oder wasserhaltigen Gemische und insbesondere Ethanol/Wasser-Gemische. Die Extraktion kann in an sich bekannter Form einer Perkolation oder einer mehrstufigen Rühr- oder Wirbelextraktion bei Raumtemperatur oder bei erhöhter Temperatur durchgeführt werden. Neben den genannten Lösungsmitteln ist in gleicher Weise auch die Extraktion mit überkritischem Kohlendioxid für die Herstellung der Rohextrakte einsetzbar.

Unter Zusatz des Trägers wird dann gegebenenfalls unter Erhöhung der Temperatur und/oder unter Vakuum das Lösungsmittel des Extraktes abgezogen und bis auf einen festen oder halbfesten Rückstand zur Trockne eingeengt.

### Beispiele:

### Bezugsbeispiel 1:

5 kg Johanniskrautextrakt (Auszugsmittel Ethanol, Gehalt 0,3 % Dianthrone, berechnet als Gesamt-Hypericin), wurden mit 2 kg Polyethylenglykol (MW 4000) gelöst bzw. suspendiert. Nach dem Auflösen des Polyethylenglykols wurde die alkoholische Phase im Vakuum eingeengt. Es entstand ein halbfester bis fester Rückstand.

Die Zusammensetzung enthielt 300 mg Johanniskrauttrockenextrakt und 200 mg des Polyethylenglykols.

### Bezugsbeispiel 2:

Analog Bezugsbeispiel 1 wurde eine Tablette aus 40 mg Gingkotrockenextrakt (1 : 50) und 110 mg Polyvinylpyrrolidon (Kollidon®25 entsprechend einem K-Wert von 25) hergestellt.

### Bezugsbeispiel 3:

1 kg Extrakt aus Cimicifuga-Wurzelstock (Auszugsmittel Ethanol), wurden mit 5 kg Polyethylenglykol (MW 4000) gelöst bzw. suspendiert. Nach dem Auflösen des Polyethylenglykols wurde die alkoholische Phase im Vakuum eingeengt. Es entstand ein halbfester bis fester Rückstand.

Die Zusammensetzung der Tablette enthielt 10 mg Trockenextrakt aus Cimicifuga-Wurzelstock und 50 mg des Polyethylenglykols.

### Beispiel 1:

Analog Bezugsbeispiel 1 wurde unter Verwendung von 40 mg Gingkotrockenextrakt (1 : 50), 20 mg des Polyvinylpyrrolidons gemäß Bezugsbeispiel 2 und 200 mg Ac-di-sol® ein fester Extrakt hergestellt, der zu einer Tablette verarbeitet wurde.

### Beispiel 2:

Analog Bezugsbeispiel 1 wurde unter Verwendung von 80 mg Crataegustrockenextrakt (4 - 7 : 1), 25 mg des Polyvinylpyrrolidons gemäß Bezugsbeispiel 2 und 200 mg Ac-di-sol® eine Kapsel hergestellt.

### Beispiel 3:

Analog Bezugsbeispiel 1 wurde unter Verwendung von 40 mg Gingkotrockenextrakt (1 : 50), 40 mg des Polyethylenglykols gemäß Bezugsbeispiel 3, 200 mg Ac-di-sol® ein Extrakt hergestellt, der zu einer Tablette verarbeitet wurde.

### Beispiel 4:

Analog Bezugsbeispiel 1 wurde unter Verwendung von 40 mg Gingkotrockenextrakt (1 : 50), 25 mg des Polyvinylpyrrolidons gemäß Bezugsbeispiel 2, 1450 mg Natriumhydrogencarbonat ein Trocken-Extrakt hergestellt, der anschließend unter Verwendung von 10 mg Aspartam®, 60 mg Polyethylenglykol mit einer Molekularmasse von 6000, 2150 mg Citronensäure und 50 mg Citronenaroma zu einer Brausetablette verpreßt wurde.

Allen vorgenannten Tabletten und Granulat-Beispielen ist die Verwendung von üblichen Tabletten- und Granulathilfsstoffen ebenso gemeinsam, wie der Einsatz von Sprengmitteln, Füllstoffen, Gleitmitteln oder Bindemitteln.

Verfahrensbedingungen zur Ermittlung von Freisetzungsraten:

Entsprechend den Vorschriften des DAB 10 V.5.4 (Wirkstoffreisetzung aus festen peroralen Arzneiformen) wurde unter Einsatz der Blattrührer-Apparatur bei folgenden Bedingungen gearbeitet: 900 ml 0,1 N/I HCI, 100 U/min, Freisetzungszeit 45 min. In allen Fällen der Beispiele 1 bis 4 wurden hervorragende ermittelt.

## Patentansprüche

1. Peroral applizierbare Arzneipflanzentrockenextrakte, von Arzneipflanzen ausgewählt aus, Passiflora, Agnus castus, Crataegus, Ginko, Brennesselextrakt, Baldrian, Cimicifuga-Wurzel oder Wurzelstock und/oder Cynara, wobei die nicht flüchtige Phase des Extraktes an einen bei Raumtemperatur festen Träger I, ausgewählt aus Polyethylenglykolen, Polyvinylalkoholen, Polyvidonacetat und/oder Polyvinylpyrrolidon, und weiterhin einen Träger II, ausgewählt aus alkoholunlöslichen, wasserunlöslichen, in Wasser quellbaren, bei Raumtemperatur festen Trägern oder Erdalkalimetall- und/oder Alkalimetallcarbonaten einschließlich Hydrogencarbonaten in mikrodisperser Form und/oder in Form einer halbfesten oder festen Lösung gebunden ist, gegebenenfalls neben weiteren Hilfs- und/oder Zusatzstoffen

2. Extrakte nach Anspruch 1, **dadurch gekennzeichnet, dass** das Polyethylenglykol eine Molekularmasse von wenigstens 1000 aufweist.

3. Extrakte nach Anspruch 2, **dadurch gekennzeichnet, dass** das Gewichtsverhältnis von Träger II zu Träger I 30 : 1 bis 1 : 1, insbesondere 10 : 1 bis 3 : 1 beträgt.

4. Extrakte nach einem der Ansprüche 1 bis 3, **dadurch gekennzeichnet, dass** das Gewichtsverhältnis von nicht flüchtigen Extraktbestandteilen zu Träger 1 : 10 bis 1 : 0.25, insbesondere 1 : 5 bis 1 : 0,6, besonders bevorzugt 1 : 3 bis 1 : 0,7 beträgt.

5. Extrakte nach einem der Ansprüche 1 bis 4, **dadurch gekennzeichnet**, das die Hilfs- und/oder Zusatzstoffe ausgewählt sind aus Bindemitteln, Gleitmitteln, Füllstoffen, Emulgatoren, Netzmitteln und/oder Sprengmitteln.

6. Extrakte nach einem der Ansprüche 1 bis 5, umfassend Kapseln, Tabletten, insbesondere Brausetabletten, Filmtabletten, Trinktabletten, Granulate, Pastillen und Dragees.

## Claims

1. Perorally administrable medicinal plant dry extracts of medicinal plants selected from *Passiflora*, chaste-tree (*vitex agnus-castus*), *Crataegus,* ginkgo, nettle extract, valerian, *Cimicifuga* root or rootstock and/or *Cynara*, wherein the non-volatile phase of the extract is bound to a carrier I being solid at room temperature which is selected from polyethylene glycols, polyvinyl alcohols, polyvidone acetate and/or polyvinyl pyrrolidone and furthermore a carrier II selected from carriers which are insoluble in alcohol, insoluble in water, swellable in water, solid at room temperature, or alkaline earth metal and/or alkali metal carbonates including hydrogencarbonates in a microdispersed form and/or in the form of a semisolid or solid solution, optionally in addition to other auxiliaries and/or additives.

2. The extracts according to claim 1, **characterized in that** the polyethylene glycol has a molecular mass of at least 1000.

3. The extracts according to claim 2, **characterized in that** the weight ratio of the carrier II to the carrier I is from 30:1 to 1:1, in particular from 10:1 to 3:1.

4. The extracts according to any one of claims 1 to 3, **characterized in that** the weight ratio of the non-volatile extract components to the carrier is from 1:10 to 1:0.25, in particular from 1:5 to 1:0.6, especially preferred from 1:3 to 1:0.7.

5. The extracts according to any one of claims 1 to 4, **characterized in that** the auxiliaries and/or additives are selected from binders, lubricants, fillers, emulsifiers, wetting agents and/or disintegrants.

6. The extracts according to any one of claims 1 to 5, comprising capsules, tablets, in particular effervescent tablets, film tablets, drinking tablets, granulates, pastilles and coated tablets.

## Revendications

1. Extraits secs de plantes médicinales pouvant être administrés par voie orale, obtenus à partir des plantes médicinales choisies parmi Passiflora, Agnus cactus, Crataegus, le Gingko, un extrait d'orties, la valériane, les racines ou rhizomes de Cimicifuga et Cynara, la phase non volatile des extraits étant liée à un support I solide à la température ambiante, choisi parmi les polyéthylèneglycols, les poly(alcool vinylique), les poly(acétate de vidone) et la polyvinylpyrrolidone, et en outre à un support II choisi parmi les supports insolubles dans un alcool, insolubles dans l'eau, pouvant gonfler dans l'eau, solides à la température ambiante, et les carbonates, y compris les hydrogénocarbonates, de métaux alcalino-terreux et/ou de métaux alcalins, sous forme micro-dispersée et/ou sous forme d'une solution semi-solide ou solide, éventuellement avec d'autres adjuvants et/ou additifs.

2. Extraits selon la revendication 1, **caractérisés en ce que** le polyéthylèneglycol a une masse moléculaire d'au moins 1000.

3. Extraits selon la revendication 2, **caractérisés en ce que** le rapport en poids du support II sur le support I est de 30:1 à 1 : 1, en particulier de 10:1 à 3:1.

4. Extraits selon l'une des revendications 1 à 3, **caractérisés en ce que** le rapport en poids des constituants non volatils de l'extrait sur le support est de 1:10 à 1:0,25, en particulier de 1:5 à 1:0.6 et plus particulièrement de 1:3 à 1:0,7.

5. Extraits selon l'une des revendications 1 à 4, **caractérisés en ce que** les adjuvants et/ou additifs sont choisis parmi les liants, les lubrifiants, les charges, les émulsifiants, les mouillants et/ou les désintégrants.

6. Extraits selon l'une des revendications 1 à 5, comprenant des gélules, des comprimés, en particulier des comprimés effervescents, des comprimés enrobés, des comprimés pour solution buvable, des granulés, des pastilles et des dragées.
